Europäisches Patentamt

European Patent Office

Office européen des brevets

(19)

(11) Veröffentlichungsnummer: **0 065 190**
**B1**

(12) # EUROPÄISCHE PATENTSCHRIFT

(45) Veröffentlichungstag der Patentschrift:
10.10.84

(51) Int. Cl.³: **C 07 C 161/02, A 01 N 47/48**

(21) Anmeldenummer: 82103834.6

(22) Anmeldetag: 05.05.82

(54) **Verfahren zur Herstellung von Jodmethylthiocyanat.**

(30) Priorität: 14.05.81 DE 3119202

(43) Veröffentlichungstag der Anmeldung:
24.11.82 Patentblatt 82/47

(45) Bekanntmachung des Hinweises auf die Patenterteilung:
10.10.84 Patentblatt 84/41

(84) Benannte Vertragsstaaten:
AT BE CH DE FR GB IT LI LU NL SE

(56) Entgegenhaltungen:
DE - A - 1 617 960
DE - B - 1 188 858
FR - A - 1 545 133
US - A - 3 361 621

(73) Patentinhaber: BASF Aktiengesellschaft,
Carl-Bosch-Strasse 38, D-6700 Ludwigshafen (DE)

(72) Erfinder: Ruland, Alfred, Dr., Schriesheimer Strasse 11,
D-6945 Hirschberg (DE)
Erfinder: Reuther, Wolfgang, Dr., Am Pferchelhang 16,
D-6900 Heidelberg (DE)
Erfinder: Pommer, Ernst-Heinrich, Dr., Berliner Platz 7,
D-6703 Limburgerhof (DE)

**Beschreibung**

Die vorliegende Erfindung betrifft ein Verfahren zur Herstellung von Iodmethylthiocyanat, z. B. durch Umsetzung von Chlormethylthiocyanat mit Natriumiodid.

Es ist bekannt, Chlormethylthiocyanat und Brommethylthiocyanat als Fungizide zu verwenden (DE-AS 1 188 858, FR 1 545 133, JP 76 06 977 und US 3 361 621).

Die Synthese der bekannten Verbindungen erfolgt üblicherweise durch Umsetzung von Ammonium- bzw. Alkalimetallthiocyanaten mit Methylenhalogeniden wie $CH_2Cl_2$, $CH_2Br_2$ bzw. $CH_2ClBr$ in geeigneten Lösungsmitteln wie Ethylenglykol, Dimethylformamid, Dimethylsulfoxid, oder in überschüssiger Ausgangsverbindung. Darüber hinaus sind Verfahren bekannt, die in Gegenwart von Phasentransferkatalysatoren, z. B. Triethylbenzylammoniumchlorid durchgeführt werden und dadurch zu besseren Ausbeuten gelangen. Andere Verfahren beruhen auf Umsetzungen halogenierter Sulfonsäurechloride mit Alkalimetallcyaniden oder Reaktionen entsprechender Sulfonsäurechloride mit Formamid (DE-AS 1 157 603).

Es wurde nun gefunden, daß man Iodmethylthiocyanat der Formel $ICH_2SCN$ erhält, wenn man Brom- oder Chlormethylthiocyanat mit einem anorganischen Iodid vorzugsweise Natriumiodid in einem organischen Lösungsmittel, z. B. Aceton, Methyl-isobutylketon, Methylethylketon, Methanol oder Ethanol, vorzugsweise Aceton, bei Temperaturen zwischen 10 und 100° C, vorzugsweise 50 bis 60° C umsetzt. Man erhält so Iodmethylthiocyanat in sehr guter Ausbeute von 80 bis 90% d. Th.

Beispiel 1

In 1500 ml Aceton wird bis zur Sättigung bei 35° C Natriumiodid (etwa 420 g) gelöst. Dazu gibt man 215 g (2 Mol) Chlormethylthiocyanat und rührt 8 Stunden zwischen 50 und 60° C. Anschließend läßt man abkühlen, destilliert im Vakuum das Aceton weitgehend ab, löst den Rückstand in 500 ml Toluol, filtriert von ungelösten Natriumiodid ab und destilliert die Lösung im Vakuum.

Ausbeute: 338 g (85% d. Th.) Iodmethylthiocyanat.
Sdp.: 53° C/0,3 mbar.

Elementaranalyse $C_2H_2INS$ (189,9):

|      | C    | H   | I    | N   | S    |
|------|------|-----|------|-----|------|
| ber. | 12,1 | 1,0 | 63,8 | 7,0 | 16,1 |
| gef. | 12,1 | 1,0 | 63,7 | 7,2 | 16,6 |

Der Wirkstoff eignet sich insbesondere zum Schutz von verschiedenen Materialien gegen den Abbau bzw. die Zerstörung durch Bakterien und Pilze. Der Wirkstoff hat auch eine starke fungitoxische und bakterizide Wirksamkeit gegenüber phytopathogenen Mikroorganismen, insbesondere gegenüber solchen, die im Boden vorkommen und Auflauf- bzw. Keimlingskrankheiten verursachen.

**Patentanspruch**

Verfahren zur Herstellung von Iodmethylthiocyanat, dadurch gekennzeichnet, daß man Chlor- oder Brommethylthiocyanat in einem organischen Lösungsmittel mit einem anorganischen Iodid bei einer Temperatur von 10 bis 1000° C umsetzt.

**Claim**

A process for the preparation of iodomethyl thiocyanate, wherein chloro- or bromomethyl thiocyanate is reacted with an inorganic iodide in an organic solvent at a temperature of from 10 to 100° C.

**Revendication**

Procédé de préparation de thiocyanate de iodométhyle, caractérisé par le fait qu'on fait réagir du thiocyanate de bromométhyle, dans un solvant organique, avec un iodure inorganique, à une température de 10 à 100° C.